# EUROPÄISCHE PATENTSCHRIFT

(11) **EP 1 319 655 B1**
(45) Veröffentlichungstag und Bekanntmachung des Hinweises auf die Patenterteilung: **02.05.2007**
(21) Anmeldenummer: 02026860.3
(22) Anmeldetag: 02.12.2002
(51) Int. Cl.: C07C 263/10, C07C 265/14

(54) **Verbessertes Verfahren für die Herstellung von (Poly-)isocyanaten in der Gasphase**
Improved process for the preparation of (poly-)isocyanates in the gaseous phase
Procédé amelioré de préparation de (poly-)isocyanates en phase gazeuze

(30) Priorität: 14.12.2001 DE 10161384
(43) Veröffentlichungstag der Anmeldung: 18.06.2003
(73) Patentinhaber: Bayer MaterialScience AG, 51368 Leverkusen (DE)
(72) Erfinder: Friedrich, Martin, Dr., 51069 Köln (DE); Stutz, Herbert, Dr., 41541 Domagem (DE)

(56) Entgegenhaltungen:
- EP-A- 0 289 840
- EP-A- 0 570 799
- EP-A- 0 676 392
- EP-A- 0 749 958
- US-A- 5 391 683

## Beschreibung

Die vorliegende Erfindung betrifft ein verbessertes Verfahren zur Herstellung von (Poly-)isocyanaten in der Gasphase mit optimierter Vermischung der Reaktanden.

Es ist bereits bekannt, dass bei Gasphasenreaktionen die gute Vermischung der Reaktanden eine wichtige Rolle bei der Erzielung hoher Umsätze und Selektivitäten spielt. Beispiele sind die Gasphasenphosgenierung von aromatischen oder (cyclo-)aliphatischen polyfunktionellen Aminen. Bei einem kontinuierlichen Verfahren werden üblicherweise die Edukte gasförmig in einen Reaktor eingebracht, wie dies in verschiedenen Patentanmeldungen beschrieben ist (z.B. EP-A 676 392, EP-A 570 799, EP-A 289 840, EP-A 749 958). Die Vermischung der Reaktionspartner soll innerhalb einer Zeit von bis zu 0,5 Sekunden bis zu einem Segregationsgrad von 10⁻³ erfolgen. Segregationsgrad ist ein Maß für die Unvollständigkeit der Vermischung (EP-A 570 799).

Die Methoden zur Durchführung kurzer Mischzeiten sind im Prinzip bekannt. Geeignet sind Mischaggregate mit bewegten oder statischen Mischorganen. Bevorzugt sind statische Mischorgane. Für die Konstruktion statischer Mischorgane ist eine Reihe verschiedener Realisierungsmöglichkeiten denkbar, z. B. die Verwendung von aus der Verbrennungstechnik bekannten Düsen, Glattstrahldüsen oder Venturidüsen.

Nachteile vieler Konstruktionen sind hoher Druckverlust oder eine Anordnung, die zu nicht ausreichend schneller Vermischung oder zu Rückvermischung in der Mischzone selbst oder im Reaktionsraum führt. Hoher Druckverlust im Mischorgan bedingt einen erhöhten Aufwand bei der Vorbereitung der gasförmigen Edukte. Mit höherem Druck ist auch eine erhöhte Siedetemperatur verbunden. Dies kann beim Verdampfen der Edukte thermische Schädigung und damit Bildung von Nebenprodukten zur Folge haben. Nicht ausreichend schnelle Vermischung oder Rückvermischung können zu erhöhter Verweilzeit eines Teils der Edukte und Produkte und damit zu unerwünschten Parallel- oder Folgereaktionen führen. Eine andere Folge unzureichender Vermischung ist unter Umständen eine ungleichmäßige Temperaturverteilung im Reaktor. Damit kann es Stellen im Reaktor geben, die übermäßig heiß sind, was zu einer verstärkten thermischen Zersetzung der Produkte führt. Zersetzungsprodukte bilden festen Rückstand, der sich an der Reaktorwand ablagert. In diesem Fall ist es üblich, den Reaktor mit einem Inliner auszurüsten, der bei Verkrustung ausgewechselt werden kann, so dass die Reinigung des Reaktors wesentlich erleichtert ist.

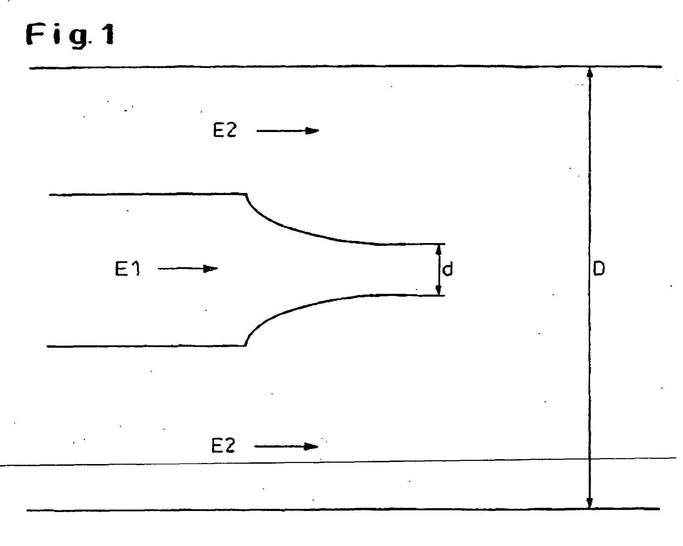

Es wurde nun gefunden, dass die bekannten Nachteile minimiert werden können, wenn man als Mischorgan eine Düse mit genau spezifizierten Maßen verwendet, die koaxial in ein Rohr eingebaut ist, das unmittelbar in den Reaktionsraum mündet. Die Vermischung der Edukte findet unmittelbar hinter dem Düsenaustritt statt. Figur 1 stellt ein erfindungsgemäßes Mischorgan mit Düse schematisch dar. Bei diesen Mischungen erfolgt die Anordnung derart, dass zwei gasförmige Edukte zur Reaktion gebracht werden und diese parallel einen Reaktor des Durchmessers D (s. Figur 1) durchströmen. Dabei wird ein Edukt E1 (s. Figur 1) über eine Düse mit dem Öffnungsdurchmesser d zentral in einen Strom des Eduktes E2 (s. Figur 1) eingebracht (wobei die Gasgeschwindigkeit von E1 groß ist gegenüber der Gasgeschwindigkeit von E2).

Es wurde gefunden, dass es eine spezifische Geometrie von Mischorgan und Reaktor, eine Düsenanordnung mit einem bestimmten Durchmesserverhältnis d/D (s. Figur 1) gibt, bei der die Einflüsse aus Druckverlust im Strom des eingedüsten Eduktes und Mischungsgüte in der Reaktionszone einen optimalen Betrieb des Reaktors gestatten. Für die Gasphasenreaktion von Aminen mit Phosgen zu Isocyanaten im erfindungsgemäßen Verfahren hat sich ergeben, dass die Zuführung von Amin zentral in einen Phosgenstrom über eine Düse, die einen kleinsten Durchmesser von 10 bis 25 % des Durchmessers des Reaktors besitzt, besonders vorteilhaft ist.

Gegenstand der Erfindung ist ein Verfahren zur Herstellung von Diisocyanaten und Triisocyanaten der allgemeinen Formel (I)

R(NCO)ₙ (I),

in welcher
- R: für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
- n: für die Zahl 2 oder 3 steht,
durch Phosgenierung der entsprechenden Diamine und/oder Triamine der allgemeinen Formel (II)

R(NH₂)ₙ (II),

in welcher
- R: für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht,
dadurch gekennzeichnet, dass die dampfförmigen Di- und/oder Triamine, gegebenenfalls verdünnt mit einem Inertgas oder mit den Dämpfen eines inerten Lösungsmittels, und Phosgen getrennt auf Temperaturen von 200°C bis 600°C erhitzt, durch ein statisches Mischorgan mit genau spezifizierter Geometrie, wobei die beiden gasförmigen Edukte parallel einen Reaktor des Durchmessers D durchströmen, wobei ein Edukt E1 über eine Düse mit dem Öffnungsdurchmesser d zentral in einen Strom des Eduktes E2 eingebracht wird, wobei die Gasgeschwindigkeit von E1 groß ist gegenüber der Gasgeschwindigkeit von E2 und das Amin durch die Düse fließt, während Phosgen durch den Ringraum um die Düse zugeführt wird und das Verhältnis von engstem Düsendurchmesser d zu Reaktordurchmesser D im Bereich von 10 % bis 25 % liegt,gemischt und kontinuierlich in einem Reaktionsraum ohne sich bewegende Teile zur Reaktion gebracht werden.

Im erfindungsgemäßen Verfahren werden Diisocyanate und/oder Triisocyanate aus den entsprechenden Diaminen und/oder Triaminen hergestellt.

Vorzugsweise werden im erfindungsgemäßen Verfahren Diisocyanate der Formel (III)

ONC-R-NCO (III),

in welcher
- R: die bei Formel (I) angegebene Bedeutung hat,
durch Phosgenierung vorzugsweise von Diaminen der Formel (IV)

H₂N-R-NH₂ (IV),

in welcher
- R: die bei Formel (II) angegebene Bedeutung hat,
hergestellt.

Als Triisocyanat der Formel (I) wird im erfindungsgemäßen Verfahren vorzugsweise 1,8-Diisocyanato-4-(isocyanatomethyl)octan, Triisocyanatononan, TIN, hergestellt.

Typische Beispiele geeigneter aliphatischer Diamine sind z.B. in der EP-A 0 289 840, geeigneter aliphatischer Triamine in EP-A 749 958 genannt.

Bevorzugt werden Isophorondiamin (IPDA), Hexamethylendiamin (HDA) und Bis(p-aminocyclohexyl)methan.

Typische Beispiele geeigneter aromatischer Diamine sind die reinen Isomeren oder die Isomerengemische des Diaminobenzols, des Diaminotoluols, des Diaminodimethylbenzols, des Diaminonaphthalins sowie des Diaminodiphenylmethans, bevorzugt sind 2,4/2,6-Toluylendiamin-Gemische der Isomerenverhältnisse 80/20 und 65/35 oder das reine 2,4-Toluylendiamin-Isomere.

Als Triamine wird vorzugsweise 1,8-Diamino-4-(aminomethyl)octan, Triaminononan eingesetzt.

Die Ausgangsamine der Formel (II) werden vor der Durchführung des erfindungsgemäßen Verfahrens verdampft und auf 200°C bis 600°C, vorzugsweise 250°C bis 450°C erhitzt und gegebenenfalls verdünnt mit einem Inertgas wie Ne, He, Ar oder mit den Dämpfen eines inerten Lösungsmittels dem Reaktor zugeführt.

Das bei der Phosgenierung verwendete Phosgen wird vor Einspeisung in den Reaktor mit dem Durchmesser D (s. Figur 1) auf eine Temperatur von 200°C bis 600°C, vorzugsweise 250°C bis 450°C erhitzt.

Die beiden gasförmigen Edukte durchströmen parallel einen Reaktor des Durchmessers D (vergl. Figur 1). Dabei wird ein Edukt E1 (s. Figur 1) über eine Düse mit dem Öffnungsdurchmesser d (s. Figur 1) zentral in einen Strom des Eduktes E2 (s. Figur 1) eingebracht, (wobei die Gasgeschwindigkeit von E1 groß ist gegenüber der Gasgeschwindigkeit von E2). Bei dem erfindungsgemäßen Verfahren fließt das Amin durch die Düse, während Phosgen durch den Ringraum um die Düse zu geführt wird. Das Verhältnis von engstem Düsendurchmesser d zu Reaktordurchmesser D liegt im Bereich von 10 % bis 25 %.

Bei der Durchführung des erfindungsgemäßen Verfahrens liegt im allgemeinen der Druck in den Zuleitungen zum Reaktionsraum bei 200 mbar bis 3000 mbar und am Ausgang des Reaktionsraumes bei 150 mbar bis 2000 mbar, wobei durch Aufrechterhaltung eines geeigneten Differenzdruckes eine Strömungsgeschwindigkeit innerhalb des Reaktionsraumes von mindestens 3 m/s, vorzugsweise mindestens 6 m/s und besonders bevorzugt 10 m/s bis 120 m/s Sorge getragen wird. Unter diesen Voraussetzungen herrschen innerhalb des Reaktionsraumes im allgemeinen turbulente Strömungsverhältnisse vor.

Die Vorteile des erfindungsgemäßen Verfahrens sind:
(a) eine gleichmäßige Reaktionszone unter Vermeidung von hot spots,
(b) eine geringe Nebenproduktbildung und
(c) die Vermeidung von festen Ablagerungen an der Reaktorwand.

### Beispiel

### Beispiel 1

In ein Mischrohr mit nachgeschalteter Diisocyanatkondensationsstufe und dieser nachfolgenden Isocyanataufarbeitung strömen kontinuierlich durch eine Düse, die in das Mischrohr hineinragt, Isophorondiamin (IPDA), Phosgen und Stickstoff in einem molaren Verhältnis von 1:4:0,1 (s. Figur 1). Die Edukte werden getrennt voneinander in vorgeschalteten Wärmetauschern verdampft und auf eine Temperatur von 320°C gebracht. Ein Gemisch aus Stickstoff und IPDA (E1, s. Figur 1) strömt durch die Düse, Phosgen (E2, s. Figur 1) im Ringraum um die Düse. Das Verhältnis der Durchmesser von Düse und Reaktionsraum beträgt 0,18:1. Der Druck in der Reaktionszone liegt geringfügig oberhalb des Atmosphärendrucks. Die Strömungsgeschwindigkeit des Reaktionsgemisches nach der Düse beträgt ca. 20 m/s. Das Reaktionsprodukt Isophorondiisocyanat (IPDI) wird nach Verlassen des Reaktors kondensiert, damit von überschüssigem Phosgen und dem Nebenprodukt Chlorwasserstoff getrennt und anschließend einer Reinigung zugeführt. Die Ausbeute an IPDI bezogen auf das eingesetzte IPDA beträgt 98,8 % der Theorie. Die gewählte Anordnung erlaubt einen sehr gleichmäßigen Betrieb. Der Reaktor weist nach zweiwöchiger Betriebsdauer keine nennenswerte Verschmutzung auf.

## Patentansprüche

1. Verfahren zur Herstellung von Düsocyanaten und/oder Triisocyanaten der allgemeinen Formel (I)
R(NCO)ₙ (I),
in welcher
R für einen (cyclo)aliphatischen oder aromatischen Kohlenwasserstoffrest mit bis zu 15 Kohlenstoffatomen steht, mit der Maßgabe, dass zwischen zwei NCO-Gruppen mindestens 2 Kohlenstoffatome angeordnet sind und
n für die Zahl 2 oder 3 steht,
durch Phosgenierung der entsprechenden Diamine und/oder Triamine der allgemeinen Formel (II)
R(NH₂)ₙ (II),
in welcher
R für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
n für die Zahl 2 oder 3 steht,
**dadurch gekennzeichnet, dass** die dampfförmigen Di- und/oder Triamine, gegebenenfalls verdünnt mit einem Inertgas oder mit den Dämpfen eines inerten Lösungsmittels, und Phosgen getrennt auf Temperaturen von 200°C bis 600°C erhitzt, durch ein statisches Mischorgan mit genau spezifizierter Geometrie, wobei die beiden gasförmigen Edukte parallel einen Reaktor des Durchmessers D durchströmen, wobei ein Edukt E1 über eine Düse mit dem Öffnungsdurchmesser d zentral in einen Strom des Eduktes E2 eingebracht wird, wobei die Gasgeschwindigkeit von E1 groß ist gegenüber der Gasgeschwindigkeit von E2 und das Amin durch die Düse fließt, während Phosgen durch den Ringraum um die Düse zugeführt wird und das Verhältnis von engstem Düsendurchmesser d zu Reaktordurchmesser D im Bereich von 10 % bis 25 % liegt, gemischt und kontinuierlich in einem Reaktionsraum ohne sich bewegende Teile zur Reaktion gebracht werden.

2. Verfahren nach Anspruch 1, **dadurch gekennzeichnet, dass** das bei der Phosgenierung verwendete Phosgen vor Einspeisung in den Reaktor mit dem Durchmesser D (s. Figur 1) auf eine Temperatur von 200°C bis 600°C erhitzt wird.

3. Verfahren nach Anspruch 1, **dadurch gekennzeichnet, dass** Diisocyanate der Formel (III)
ONC-R-NCO (III),
in welcher
R die in Anspruch 1 bei Formel (I) angegebene Bedeutung hat,
durch Phosgenierung von Diaminen der Formel (IV),
H₂N-R-NH₂ (IV),
in welcher
R die in Anspruch 1 bei Formel (II) angegebene Bedeutung hat,
hergestellt werden.

4. Verfahren nach Ansprüchen 1 und 3, **dadurch gekennzeichnet, dass** Isophorondiamin (IPDA) oder Hexamethylendiamin (HDA) oder Bis(p-aminocyclohexyl)methan als Diamin der Formel (IV) eingesetzt wird.

5. Verfahren nach Anspruch 1, **dadurch gekennzeichnet, dass** 2,4/2,6-Toluylendiamin-Gemische der Isomerenverhältnisse 80/20 und 65/35 oder das reine 2,4-Toluylendiamin-Isomere als Diamin der Formel (IV) eingesetzt wird.

6. Verfahren nach Anspruch 1, **dadurch gekennzeichnet, dass** Triisocyanatononan (TIN) hergestellt wird.

## Claims

1. Process for the production of diisocyanates and/or triisocyanates of the general formula (I)
R(NCO)ₙ (I)
where
R represents a (cyclo)aliphatic or aromatic hydrocarbyl radical having up to 15 carbon atoms, with the proviso that there are at least 2 carbon atoms arranged between two NCO groups and
n represents the number 2 or 3
by phosgenation of the corresponding diamines and/or triamines of the general formula (II)
R(NH₂)ₙ (II)
where
R represents a (cyclo)aliphatic or aromatic hydrocarbyl radical having up to 15 carbon atoms, preferably 4 to 13, with the proviso that there are at least two carbon atoms arranged between two amino groups and
n represents the number 2 or 3
**characterized in that** the vaporous di- and/or triamines, optionally diluted with an inert gas or with the vapours of an inert solvent, and phosgene are separately heated to temperatures of 200°C to 600°C, mixed by a static mixing element having a precisely specified geometry, the two gaseous reactants flowing parallel through a reactor of diameter D, one reactant E1 flowing through via a nozzle of opening diameter d to flow centrally into a stream of reactant E2, the gas velocity of E1 being high compared with the gas velocity of E2 and the amine flows through the nozzle whereas phosgene is supplied through the annular space around the nozzle and the ratio of narrowest nozzle diameter d to reactor diameter D being in the range from 10% to 25%, and continuously reacted in a reaction space without moving parts.

2. Process according to Claim 1, **characterized in that** the phosgene used in the phosgenation is heated to a temperature of 200°C to 600°C before being fed into the reactor of diameter D (see Figure 1).

3. Process according to Claim 1, **characterized in that** diisocyanates of the formula (III)
ONC-R-NCO (III)
where
R has the meaning stated in Claim 1 for the formula (I),
are produced by phosgenation of diamines of the formula (IV)
H₂N-R-NH₂ (IV)
where
R has the meaning stated in Claim 1 for the formula (II).

4. Process according to Claims 1 and 3, **characterized in that** isophoronediamine (IPDA) or hexamethylenediamine (HDA) or bis(p-aminocyclohexyl)methane is used as diamine of the formula (IV).

5. Process according to Claim 1, **characterized in that** 2,4/2,6-tolylenediamine mixtures having 80/20 and 65/35 isomer ratios or the pure 2,4-tolylenediamine isomer is used as diamine of the formula (IV).

6. Process according to Claim 1, **characterized in that** triisocyanatononane (TIN) is produced.

## Revendications

1. Procédé de fabrication de composés diisocyanates et/ou triisocyanates de la formule générale (I)
R(NCO)ₙ (I),
dans laquelle
R représente un reste hydrocarbure (cyclo)aliphatique ou aromatique avec jusqu'à 15 atomes de carbone, avec la condition qu'au moins 2 atomes de carbone sont disposés entre deux groupes NCO et
n représente le chiffre 2 ou 3,
par phosgénation des diamines et/ou triamines correspondantes de la formule générale (II)
R(NH₂)ₙ (II),
dans laquelle
R représente un reste hydrocarbure (cyclo)aliphatique ou aromatique avec jusqu'à 15, de préférence de 4 à 13, atomes de carbone, avec la condition qu'au moins 2 atomes de carbone sont disposés entre deux groupes amines et
n représente le chiffre 2 ou 3,
**caractérisé en ce que** les diamines et/ou triamines gazeuses, le cas échéant diluées avec un gaz inerte ou avec les vapeurs d'un solvant inerte, et le phosgène sont chauffés séparément à des températures de 200°C à 600°C, sont mélangés dans un organe de mélange statique de géométrie exactement spécifiée et mis en réaction en continu dans un espace de réaction sans pièces mobiles, les deux éduits gazeux circulant en parallèle dans un réacteur de diamètre D, un éduit E1 étant introduit de manière centrale via une tuyère de diamètre d'ouverture d dans un flux de l'éduit E2, la vitesse du gaz de E1 étant grande par rapport à la vitesse du gaz de E2 et l'amine circulant à travers la tuyère pendant que le phosgène est introduit à travers l'espace annulaire autour de la tuyère et le rapport du diamètre de tuyère le plus étroit d sur le diamètre du réacteur D étant de l'ordre de 10 % à 25 %.

2. Procédé suivant la revendication 1, **caractérisé en ce que**, avant l'introduction dans le réacteur de diamètre D (voir figure 1), le phosgène utilisé lors de la phosgénation est chauffé à une température de 200°C à 600°C.

3. Procédé suivant la revendication 1, **caractérisé en ce qu'**on fabrique des diisocyanates de la formule (III)
ONC-R-NCO (III)
dans laquelle
R a la signification indiquée dans la revendication 1 pour la formule (I),
par phosgénation de diamines de la formule (IV),
H₂N-R-NH₂ (IV),
dans laquelle
R a la signification indiquée dans la revendication 1 pour la formule (II).

4. Procédé suivant les revendications 1 et 3, **caractérisé en ce que** l'isophoronediamine (IPDA) ou l'hexaméthylènediamine (HDA) ou le bis(p-aminocyclohexyl)méthane sont utilisés comme diamines de la formule (IV).

5. Procédé suivant la revendication 1, **caractérisé en ce qu'**on utilise des mélanges de 2,4-toluylènediamine / 2,6-toluylènediamine des rapports d'isomères 80/20 et 65/35 ou l'isomère pur de 2,4-toluylènediamine comme diamine de la formule (IV).

6. Procédé suivant la revendication 1, **caractérisé en ce qu'**on fabrique du triisocyanatononane (TIN).
